# EUROPEAN PATENT SPECIFICATION

(11) **EP 1 111 051 B1**
(45) Date of publication and mention of the grant of the patent: **23.02.2005**
(21) Application number: 99310311.8
(22) Date of filing: 21.12.1999
(51) Int. Cl.: C12N 15/29, C12N 15/82, A01H 1/00

(54) **Promoter for guard cell-specific gene expression in plants**
Promoter für eine schliesszellspezifische Genexpression in Pflanzen
Promoteur pour l'expression spécifique de géne dans de cellules stomatiques de plantes

(43) Date of publication of application: 27.06.2001
(73) Proprietor: Korea Kumho Petrochemical Co. Ltd., Chongno-Gu, Seoul (KR)
(72) Inventor: Kim, Yong-Sig, Kwangsan-gu, Kwangju-city (KR); Park, Sun-Choong, Kwangsan-gu, Kwangju-city (KR); Cho, Jeong-Woo, Kwangsan-gu, Kwangju-city (KR); Chung, Chang-Ho, Seo-gu Kwangju-city (KR)
(74) Representative: Nash, David Allan

(56) References cited:
- WO-A-93/18169
- BEVAN ET AL.: "Arabidopsis thaliana DNA chromosome 4, BAC clone T19F6, partial sequence (ESSA project)" EMBL SEQUENCE DATABASE, 29 July 1999 (1999-07-29), XP002137840 HEIDELBERG DE

## Description

### BACKGROUND OF THE INVENTION

### Field of invention

The present invention relates to an expression cassette and plasmids comprising a promoter, which ensures a specific gene expression in the stomatal guard cell of plants and no expression in mesophyllic cells or epidermal cells. The present invention further relates to a process for the preparation of transgenic plant cells, which contain sequences of the expression cassette as well as the use of the plasmids containing expression cassette for the preparation of transgenic plants.

### Description of the related art

With the recent development in biotechnology such as genetic engineering and cell technology, a system for directly introducing a desirable gene into plants is being established even for crop plants, and is expected to be one capable of overcoming the problems in the conventional breeding. In this where a foreign gene is precisely introduced into a plant, the gene can be sufficiently expressed in the intended tissue in good time. For this purpose, a tissue-specific promoter must be linked to the foreign gene to thereby make the gene expressible under the control of such promoter.

The aerial portions of plants are covered with a large number of stomatal guard cells on the surface of green leaves that is believed to play an important role in CO₂ exchange and also in protecting these organisms from water loss, UV irradiation and frost damage. In recent years stomatal guard cells have become the focus of considerable attention by investigators interested in studying stimulus response coupling in higher plants. This popularity reflects the fact that these cells respond in a quantifiable manner to a range of important environmental variables and are tractable to the gamut of single cell-based techniques and ultimately to understand how extracellular stimuli control plant growth and development. A promoter from the ADP glucose pyrophosphorylose gene of solanum fuberosum, which specifically directs expression to the guard cells, is known from WO 9 318 169.

In order to understand the regulatory role of the promoter of *Arabidopsis thaliana* trehalase gene (EMBL Ac. No. AL 109 619) and to determine if said promoter direct guard cell specificity to the desired genes, we isolated the promoter of Arabidopsis trehalase gene. We also tested DNA construct comprising said promoter operably linked to a reporter gene, β-glucuronidase (hereinafter, named as GUS reporter gene), by transformation of plant cell culture and the subsequent generation of stable transgenic plants. Tissue specificity and a precise developmental pattern of trehalase gene promoter-driven GUS gene expression in stable transgenic Arabidopsis was observed in stomatal guard cells, and without any detectable expression in mesophyllic and epidermis cells.

### SUMMARY OF THE INVENTION

It is an object of the present invention to provide DNA molecule which represent genes or fragments thereof which are expressed in guard cell-specific manner.

It is also an object of the present invention to provide DNA molecule representing a promoter of a gene which is expressed in guard cell-specific manner.

It is yet another object of the invention to provide a DNA molecule capable of directing reporter or desired gene expression to guard cells of plants.

It is also an object of the invention to provide a reporter construct useful for testing the ability of said promoter to drive expression of a reporter gene in guard cell-specific manner in plants.

It is yet another object of the invention to provide a DNA molecule which direct desired gene expression in guard cells of plants resulting in enhancing photosynthetic assimilation rate, resistance against pathogens and adverse weather conditions or confer a growth advantage to plants.

It is also an object of the invention to provide a method for generating a transgenic plant transformed with guard cell-specific expression vector that directs expression of desired genes in guard cells of plants.

According to the present invention, a desired foreign gene and a terminator therefore can be linked to the downstream site of the promoter obtained, and introduced into plant such as dicots and monocots, whereby the foreign gene can be expressed in the plant stomatal guard cells. Therefore, the present invention can be utilized to drive guard cell-specific gene expression in plants for resistance to adverse weather conditions including but not limited to cold, drought, salt, heat and flooding as well as genes which influence growth of or yield from said plants. Therefore, it is considered important by those skilled in the art to provide guard cell-specific gene expression manner, and, as a result, have completed the present invention.

With the present invention it is possible to regulate the transpiration process and the gas exchange of a plant in regard to the increase of yield through the manipulation of genes which are expressed specifically in stomatal guard cells.

An increase in yield of crop plants can be achieved by regulating either the exchange of gases for the high photosynthetic assimilation or the stress-related inhibition of growth. The use of known expression system is not appropriate for the productivity of the plant because of the broad-ranging consequences of a non-guard cell specific gene expression in plants. Regarding to this, the guard cells of the epidermis of the leaf tissue are a valuable starting point.

A pair of specialized epidermal cells, so named as the guard cells exerts the plant's primary means of regulating the exchange of gases across the leaf surfaces, which surround the stomatal pore. Guard cells control the gas exchange between carbon dioxide uptake and transpiration. Besides the concentration of carbon dioxide, a constituent of the control system is the concentration of dissolved substances since these regulate stomatal apertures.

Water stress affects both stomatal conductance and photosynthetic activity in plants. Upon stomatal closure during early stages of water stress, water-use efficiency may taken up more CO₂ per unit of water transpired because stomatal closure inhibits transpiration rather than decreasing intercellular CO₂ concentrations and dehydration of mesophyll cells inhibit photosynthesis. Stomatal limitations to photosynthesis can be overcome by a higher external concentration of CO₂, but any direct effect of water stress on mesophyll metabolism will not be removed by exposure to high concentrations of CO₂. Since such changes in the guard cells have wide-reaching consequences for the metabolism of plants, it is desirable to allow these changes only in certain areas of plants or during a desired time period in the plant growth cycle. For this reason, there is a great interest in the identification and the economic use of expression system, which are responsible for the specific transcription in guard cells.

In order to specify such promoter(s) or regulatory DNA sequences, one must first seek products that appear only in a certain plant tissues such as in guard cells or at certain times in the plant growth. Once the responsible gene is identified and isolated, it is necessary to have a basic investigation of the sequence and above all the identification and isolation of the desired transcriptional regulatory region.

The present inventors have conducted extensive researches and found a promoter capable of functioning in plant cells, which enables tissue-specific expression of desired genes in stomatal guard cells, thereby completed the present invention.

With the help of guard cell specific promoter, by the expression of desired genes, the temporal regulation of the opening period of guard cells or the osmotic adjustment of guard cells, etc. can be achieved. The present invention provides a guard cell specific promoter and expression vector with which such effects are possible.

There is now provided an expression cassette whose DNA sequence comprises a promoter for guard cell-specific gene expression that causes no expression in mesophyllic cells or epidermal cells. With said expression cassette, a gene manipulation of plants is possible which causes the temporal regulation of the opening period of guard cells or the osmotic adjustment of guard cells, etc.

On the expressions cassette there is located the DNA sequence of the guard cell specific promoter having the sequence (Seq. ID NO:1 in SEQUENCE LISTING).

Therefore, the present invention provides:
1. An isolated promoter, which leads to specific expression of desired DNA fragment only in plant stomatal guard cells and not in mesophyllic or epidermal cells of transformed plants, comprising a nucleotide sequence of about 2 Kb of SEQ ID NO: 1,
2. A cloning vector pLES98061 (KCTC 0663BP) comprising the promoter of SEQ ID NO: 1,
3. An expression vector pLES98062 (KCTC 0664BP) comprising the promoter of SEQ ID NO: 1.

### BRIEF DESCRIPTION OF THE FIGURES

Fig. 1 shows the nucleotide sequence and restriction site of trehalase gene promoter from *Arabidopsis thaliana.* The heat shock responsive element is in shaded box, and the stress-responsive elements are underlined.
Fig. 2a shows pLES98061 as a cloning vector, which is the plasmid comprising said promoter without GUS fusion of the present invention and Fig. 2b shows pLES98062 as an expression vector, which is the plasmid comprising said promoter with GUS fusion of the present invention.
Fig. 3 shows GUS activity detected by histochemical assay. Fig. 3a shows Leaf tissue detached from wild type of Arabidopsis, Fig. 3b shows leaf tissue detached from Arabidopsis transformed with said promoter-GUS construct (pLES98062). GUS expression is detectable only in the guard cell-specific manner but not in the mesophyllic cells or epidermis cells.

### DETAILED DESCRIPTION OF PREFERRED EMBODIMENTS

The present invention provides a promoter useful for directing direct guard cell-specific gene expression in plants. The promoter is preferably utilized to drive expression of desired gene encoding gene product that confers a selective advantage upon a plant in which said gene product is expressed. There exists a need in the art for transcriptional regulatory region of plant genes which drive expression of the genes specifically within certain target site of plants. Such transcriptional units are defined within the present invention to function in a "guard cell-specific" manner. The present invention relates to the isolation, characterization and utilization of the promoter and expression vector for the gene expression in guard cell-specific manner. GUS staining assay reveals that the promoter selectively drive the expression of genes in guard cells of plants.

Within the present invention, a guard cell-specific promoter is defined as an element involved in regulating the transcription of a desired gene to the guard cells. The promoter may alternatively be referred to as a gene promoter.

A gene product that confers a selective advantage to plants is defined as any gene product which, upon expression in the plant, confers increased growth rate, yield of product or enhancing photosynthetic assimilation rate, the temporal regulation of gas exchanges, or resistance to threats to said plant's ability to thrive including but not limited to pathogens and adverse weather conditions relative to plants that do not express said gene product.

An expression vector is defined as a DNA construct comprising at least one gene which, upon transcription into a cell, results in expression of the product of the gene under the control of the promoter.

In one embodiment, the present invention comprises a DNA molecule comprising a promoter that is useful in directing guard cell-specific gene expression.

In another embodiment, the present invention comprises a DNA molecule comprising a guard cell-specific gene promoter operably linked to a reporter or desired genes.

In another embodiment, the present invention comprises expression vector comprising guard cell-specific gene promoter operably linked to desired genes, expression of the genes within a plant conferring a selective advantage to the plants.

In the present invention conventional genetic engineering methods are described, for example, in Jose M. Martinez-Zapater and Julio Salinas, Arabidopsis protocols, Humana press, 1998; J. Sambrook et al., Molecular cloning, 2^{nd}. Ed., Cold Spring Harbor Laboratory press, 1989; and so can be used.

Description will be made to the promoter which is functional in plant cells comprising a nucleotide sequence of SEQ ID NO: 1 and have the following properties:
1) isolated from *Arabidopsis thaliana*;
2) comprising restriction enzyme sites for BstSFI, BamHI, BsaWI, HindIII, Xbal, BglII, EcoRI, BsrDI and SmaI; and
3) comprising a heat-shock responsive element and a couple of stress-responsive elements in a nucleotide sequence of SEQ ID NO: 1.

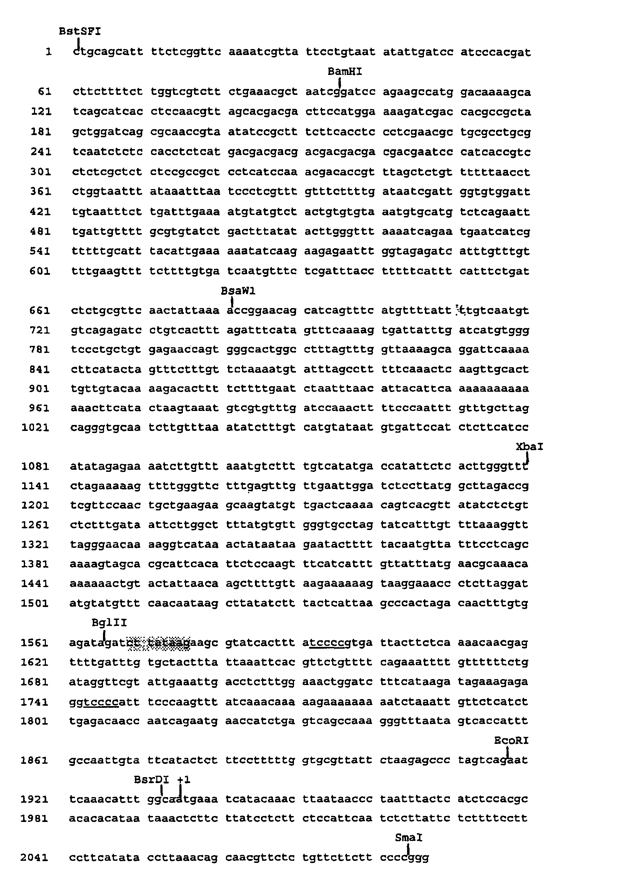

The present invention provides:
1) The first aspect of the present invention is a gene expression regulatory DNA that comprises a promoter derived from Arabidopsis trehalase gene enabling the expression of desired gene in plant stomatal guard cells and for regulating the expression of the structural gene based on the promoter. The promoter includes one which comprises cleavage sites to be cleaved with restriction enzymes, BstSFI, BamHI, BsaWI, HindIII, XbaI, BglII, EcoRI, BsrDI and SmaI, in that order (Fig. 1) and which comprises a gene substantially having the nucleotide sequence of SEQ ID NO: 1 in Sequence Listing. Furthermore, the promoter comprises a heat-shock element and two stress-responsive elements. Also, the promoter may be composed of a base sequence substantially having the sequence of SEQ ID NO: 1 with some bases deleted, inserted or substituted, so far as the resulting sequences effectively retain the promoter and expression regulatory activities. In addition, these sequences having some bases deleted, inserted or substituted are essentially the same in function as the base sequences, so far as they effectively retain the promoter activity.
2) The second aspect of the present invention is an expression cassette or a vector integrating expression cassette to be used as an expression vector.
3) The third aspect of the present invention is a method for producing transgenic plants, that comprise the vector or expression cassette, by introducing it directly into a desired plant to integrate it to chromosome, etc. As plant cells to which the expression cassette or vector is introduced is preferred the plant stomatal guard cells wherein the expression of a foreign gene is specifically elevated.
4) The fourth aspect of the present invention is a transgenic plant as transformed with the vector or expression cassette. Therefore, the transduction of the expression cassette or vector into plant cells with regeneration potency may provide effective means for generating transgenic plants conferring high plant productivity.

As has been mentioned hereinabove, a guard cell-specific promoter is a gene expression regulatory DNA, being capable of specifically expressing desired genes in stomatal guard cells, derived from Arabidopsis trehalase gene.

Trehalase, which has been known to function in plants for drought stress tolerance, has been known as an enzyme usable in producing 2 molecules of glucose from trehalose. Regarding the gene of trehalase, the complete sequence of cDNA of soybean, which comprises 2123 nucleotides, has been reported, and the amino acid sequence thereof comprising 557 residues (∼56 kD) has also been deduced (Aeschbacher et al., 1999. Plant Physiol. 119:489-495). On the other hand, although cDNA comprising the entire translational region of trehalase was isolated and analyzed for its structure, there is no report referring to the isolation of the promoter gene, let alone the analysis of the nucleotide sequence thereof. Regarding the promoter region that acts to control the transcription of such trehalase gene, an effective promoter that enables tissue-specific expression, particularly in guard cells, of desired genes in order to produce desirably transformed plants has been desired.

The promoter is preferably used for stomatal guard cell-specific expression of desired genes, which mean a promoter accessing a capability of regulating expression of protein in guard cells when desired gene is ligated downstream of the promoter. Also, the promoter as described above may be further modified by deleting, inserting or substituting some bases of the promoter in regard to retain said promoter activity.

The plasmid of the present invention can be prepared by the following process.

The plasmid comprising the promoter of the present invention can be constructed to have one or more cloning sites for excising or inserting desired structural gene(s) to the downstream of the promoter of the present invention.

The desired gene can be prepared synthetically or extracted naturally or can consist of a mixture of synthetic and natural DNA constituents, which encode proteins, enzymes, carrier, pumps or phytohormones. In general, synthetic DNA sequences are produced with codons, which are preferred for plants. These preferred codons for plants could be selected from codons with the highest protein abundance, which are expressed in the most interesting plants. In the preparation of an expression cassette, various DNA fragments can be manipulated in order to obtain a gene that reads suitably in a sense orientation and which is supplied with the correct reading.

The promoter, GUS reporter gene or desired structural gene and a terminator which are functional in plant cells are inserted into a multicloning site of a binary vector, for example, pBI101.2 (Clontech, USA). Further DNA sequences which contain information for the formation and quantitative distribution of endogenous products or the formation of exogenous expression products can be fused to the promoter of the present invention.

More specifically, the plasmid also comprises chimeric gene(s) which is prepared by ligating desired structural gene(s) to the promoter for the expression of desired protein(s) in stomatal guard cells, and also which is autonomously replicating, inheritable and the like.

In the present invention, transgenic plants comprising the promoter for a guard cell specific gene expression can be prepared by the following process:
a) isolation of the guard cell-specific promoter as described in Examples 1. The clone contains an approximately 2.0 kb size DNA-fragment, that contains a guard cell specific regulator element (see Fig. 1).
b) preparation of the plasmid pLES98061 (KCTC 0663BP) and/or pLES98061-GUS (pLES98062; KCTC 0664BP), using the approximately 2.0 kb size SalI/SmaI-fragment of the regulatory starter-region of the trehalase gene of *Arabidopsis thaliana* (Example 2).
c) transfer of the T-DNA of the plasmid pLES98062 (KCTC 0664BP) in a plant cell (Example 3).

For increasing the expression efficiency of desired gene(s), plant cells are preferably transformed with a vector comprising the promoter of the present invention. Once the desired gene is integrated in plant genome, it is generally stable and remains in the offspring of the original transformed cells. The transformed cells grow within the plants in the usual manner (McCormick et al. 1986. Plant Cell Reports 5:81-84). These plants can be grown normally and crossed with plants comprising the same gene. Further two or more generations should be grown to ensure that the phenotypic properties remains stable and inherited. Also, the resulting hybrid individuals have the corresponding phenotypic properties.

All plant species are suitable as host plants for the guard cell specific expression. Suitable plants are agriculturally valuable monocotyledonous (i.e., maize, rice, barley and the like) and dicotyledonous (i.e., phaseolus beans, tomato, cucumber, potato, sweet potato, cotton, onion, citrus, pecan, lettuce, beans and the like).

Since the promoter enables expression of a desired gene in guard cells, the use of expression cassette of the present invention is especially valuable in improving the environmental stress tolerance, or creating of crop plants having increased photosynthetic assimilation through the temporal regulation of stomatal apertures.

In this invention, the present inventors have used Arabidopsis plant that has been transformed with chimeric gene constructs made up of the promoter fused to the GUS reporter gene to investigate guard cell-specific expression. Heterologous expression studies using the promoter GUS fusion show that the promoter is expressed in stomata guard cells and not in mesophyllic and neighboring cells.

The plasmid pLES98061 comprises the approximately 2.0 kb of the promoter of trehalase gene of *Arabidopsis thaliana,* a linker and a transcription terminator of the nopaline synthase gene.

All the processes necessary for the present invention and which are known per se will first of all be listed:

### 1. Isolation of guard cell-specific promoter and its cloning

Guard cell-specific promoter can be prepared from the 5'-upstream region of trehalase gene on the Arabidopsis BAC clone by standard methods, for example, according to those described in Jose M. Martinez-Zapater and Julio Salinas, Arabidopsis protocols, Humana press, 1998; J. Sambrook et al., Molecular cloning, 2^{nd} Ed., Cold Spring Harbor Laboratory press, 1989).

In addition to methods described above, the DNA can be isolated by screening chromosomal library prepared by conventional methods with probes homologous to the 5'-upstream region of trehalase gene, for example, according to those described in J. Sambrook et al., Molecular cloning, 2^{nd} Ed., Cold Spring Harbor Laboratory press, 1989.

Also, procedures related to gene cloning necessary for conducting the present invention including the digestion with restriction enzymes, DNA linking procedure, *E. coli* transformation, etc. are performed by standard methods in J. Sambrook et al., Molecular cloning, 2^{nd} Ed., Cold Spring Harbor Laboratory press, 1989.

Alternatively, the promoter can be artificially synthesized using a DNA synthesizer based on the nucleotide sequence.

### 2. Nucleotide sequencing

The promoter region can be sequenced, for example, according to Maxam-Gilbert method (Methods in Enzymology 65:499. 1980) or dideoxynucleotide chain termination method (Gene 19:269. 1982).

### 3. Construction of expression vector

Expression cassette can be prepared by linking a desired gene downstream from the promoter followed by linking nopaline synthetase gene (NOS) terminator downstream from the desired gene. Expression cassette thus may be transferred directly as such in a linear form into plant chromosomes or integrated into a described plasmid to be used as expression vector described below.

For plant transformations, the gene constructions were cloned into the binary vector pBI101.2 (Clontech). It is preferable to select plasmid with the replication origin suitable to organisms to which the expression vector is transferred. When expression vector is recovered in large quantities, it is preferable to select plasmid with large copy numbers.

### 4. Formation of Trangenic plants

As described above, transgenic plants can be created by transferring a foreign DNA integrated into expression cassette or vector into plants, forming a novel cultivar with different properties from those of wild type plant. The transformation of the plasmids into the Arabidopsis plants was carried out by means of the *Agrobacterium tumefaciens* strain LBA4404.

### 5. β-Glucuronidase-activity assay (GUS-Assay)

To determine the promoter activity of said expression cassette, a reporter gene, such as a β -glucuronidase gene (GUS) or luciferase gene, may be linked to the downstream site of the promoter region to form the expression vector. To determine the promoter activity in guard cells by the use of the reporter plasmid as constructed in the above, the GUS activity or luciferase activity in the resulting cell lines is measured.

In the present invention, the beta-glucuronidase has been used for the histochemical activity determinations. Activity measurement were carried out by the method of Jefferson et al. (1987) EMBO J 6: 3901-3907.

The following examples illustrate the preparation of the expression cassette, whose DNA sequence comprises a regulatory sequence for a guard cell specific gene expression. The introduction of the sequence in a plasmid is also illustrated in that a transformation of plant cells is possible. In addition, the regeneration of transgenic plants and the investigation of the function of the expression cassette in transgenic plants is shown.

### EXAMPLE 1

### Isolation of guard cell-specific promoter

Based on *Arabidopsis thaliana* BAC T19F06 genomic sequence which has a putative trehalase isolog, 5' end primer (5'-CTGCAGCATTTTCTCGGTTCAAAATCG - 3') and 3' end primer (5'-CCCGGGGAAGAAGAACAGAGAACGTT - 3') were generated for amplification of trehalase promoter region. Arabidopsis genomic DNA was used as a Polymerase chain reaction (PCR) template. Polymerase chain reaction was heated at 95°C for 5 min followed by 30 cycles of 30 sec at 95°C, 30 sec at 50°C, and 2 min at 72°C followed by holding at 4°C. The PCR products were analyzed with 0.8% agarose gel electrophoresis. About 2 kbp trehalase gene promoter PCR product was eluted by E.Z.N.A. agarose gel purification system. About 2.0 kbp size of trehalase gene promoter PCR product was then cloned into the SaII/SmaI restriction site of pGEM-T easy vector system by the manufacturer's guide and the Plasmid pLES98061 resulted. The pLES98061 was subjected into nucleotide sequencing reaction with ABI Prism 310 Genetic Analyzer. Sequence analysis revealed the 2087 bp nucleotides and harbored a heat shock responsive element at the position of 1568 and a two stress responsive elements at the position of 1584^{th} and 1751^{th}, respectively. The transcription start site for trehalase gene was positioned at the 1935^{th} of nucleotide sequence.

### EXAMPLE 2

### Construction of guard cell-specific expression vector

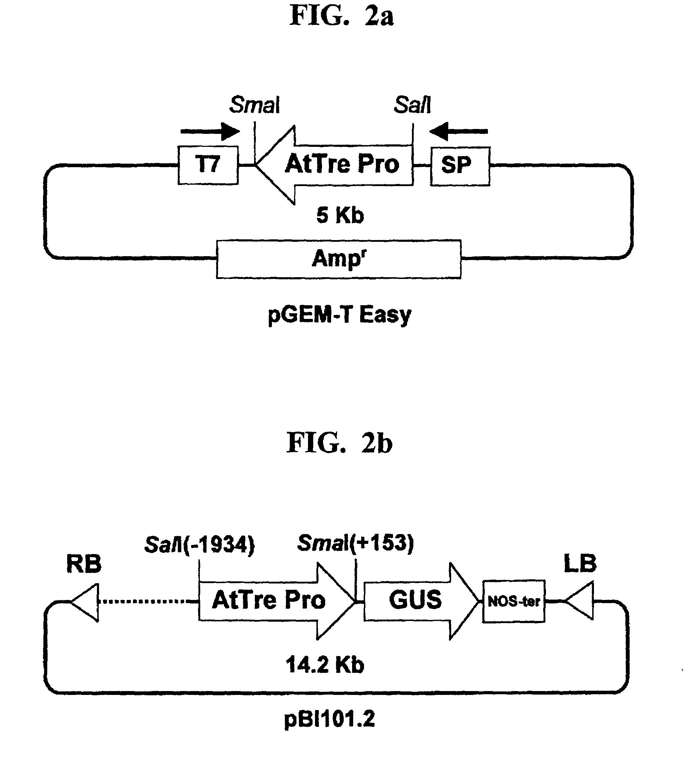

In order to generate plasmid vector for use in driving guard cell-specific gene expression in plants, a recombinant construct was engineered. About 2.0 kbp size Sal I/SmaI insertion of pLES98061 was inserted into the SaII/SmaI restriction site of pBI101.2, which contains the β-glucuronidase(GUS) coding region and the terminator of NOS. The recombinant plant expression vector, comprising trehalase gene promoter fused to GUS gene, was resulted and named as pLES98062 (Fig. 2). The construct pLES98062, with the coding region of β-glucuronidase as reporter gene, was transferred into *Agrobacteria tumefaciens* strain LBA4404 and the Agrobacteria containing the chimeric trehalase gene promoter/GUS gene is used for transformation of Arabidopsis leaves. With the help of this plasmid an expression of preferred genes under the control of trehalase gene promoter is also possible.

### EXAMPLE 3

### Transformation of Arabidopsis

*Arabidopsis thaliana* Ecotype Columbia was used as a test plant for trehalase promoter activity. Arabidopsis was transformed with *Agrobacterium tumefaciens* LBA4404 comprising pLES98062 by vacuum infiltration method as previously described in Jose M. Martinez-Zapater and Julio Salinas, Arabidopsis protocols (Humana press, 1998). Arabidopsis was grown at 16 hr light period for 4 weeks.

### EXAMPLE 4

### GUS activity assay of transformed Arabidopsis

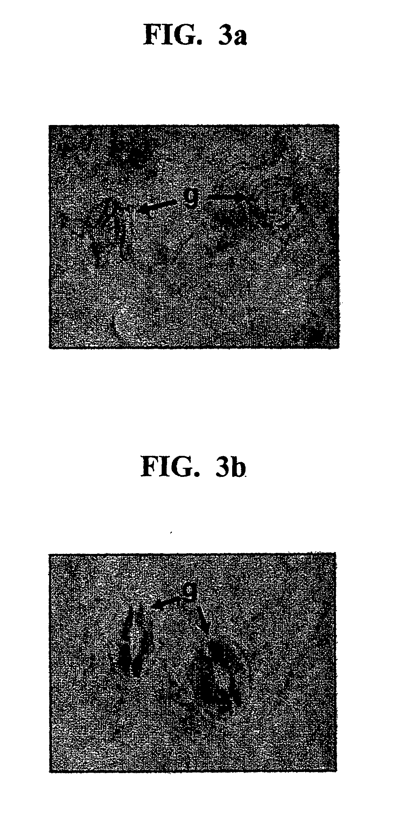

The β-glucuronidase is indicative of a histological determination of its activity and can thus be introduced for the analysis of the cell specificity of a regulatory region of an expression system to be tested. Transformed Arabidopsis plants were subjected in GUS activity assay. The full grown Arabidopsis leaves were detached and soaked into assay solution containing 100 mM sodium phosphate buffer (pH 7.0), 10 mM EDTA (pH 8.0), 0.5 mM potassium ferricyanide, 0.5 mM potassium ferrocyanide, and 0.1% Triton X-100, 0.3% 5-bromo-4-chloro-3-indolyl glucuronide (X-Gluc). After overnight incubation at 37°C, the leaves were transferred into 100% ethanol solution to decolorize the leaf chlorophyll. GUS stained leaves were examined under the microscopy. Of twelve resulting independent transformants, it is clearly shown that the 2.0 kbp size SaII/SmaI fragment of trehalase gene promoter/GUS has a comparable activity in the stomata guard cells of leaves (Fig. 3), while for mesophyllic and epidermis cells of Arabidopsis plants and roots no activity was seen. Therefore, the guard cell-specific pattern of trehalase gene promoter/GUS expression suggests that 2.0 kbp size SalI/SmaI fragment of trehalase gene promoter may be useful for directing expression of desired genes for enhanced photosynthetic assimilation rate, pathogen resistance, tolerance against adverse weather conditions or a growth advantage to plants.

### DEPOSIT OF BIOLOGICAL MATERIAL

### INTERNATIONAL DEPOSITARY AUTHORITY:

Korean Collection for Type Cultures
Korean Research Institute of Bioscience and Biotechnology (KRIBB),
#52, Oun-dong, Yusong-ku,
Taejon 305-333
Republic of Korea

| **ACCESSION NUMBERS:** | |
|---|---|
| **Microorganism (Applicant's Reference)** | **Accession Number** |
| *Escherichia coli* XL1-Blue/pLES98061 | KCTC 0663BP |
| *Agrobacterium tumefaciens* LBA4404/pLES98062 | KCTC 0664BP |

## Claims

1. An isolated promoter, which leads to specific expression of a desired DNA fragment only in plant stomatal guard cells and not in mesophyllic or epidermal cells of leaves of transformed plants, comprising the base sequence shown in SEQ ID NO: 1, or a base sequence substantially having the nucleotide sequence of SEQ ID NO:1 which retains the promoter function of the base sequence of SEQ ID NO: 1.

2. A promoter according to claim 1, wherein said promoter comprises a nucleotide sequence of 2.0 Kb having the following characteristics:
i) isolated from *Arabidopsis thaliana*;
ii) having restriction enzyme sites for BstSFI, BamHI, BsaWI, HindIII, XbaI, BgIII, EcoRI, BsrDI and SmaI; and
iii) comprising a heat-shock responsive element and two stress-responsive elements.

3. A promoter according to claim 1, wherein said promoter is the 5' transcriptional regulatory region of a gene coding for trehalase.

4. A cloning vector, wherein said promoter is contained in the plasmid pLES98061 and deposited as KCTC 0663BP, comprising a promoter as defined in claim 1.

5. An expression vector comprising a promoter as defined in claim 1, wherein said promoter is fused to the GUS reporter gene or a desired structural gene contained in the plasmid pLES98062, deposited as KCTC 0664BP.

6. A microorganism containing the plasmid according to claim 4, wherein said microorganism is *E. coli* XL1-Blue/pLES98061 strain.

7. A microorganism containing the chimeric gene according to claim 5, wherein said microorganism is *Agrobacterium tumefaciens* LBA4404/pLES98062 strain.

8. A method for the production of transgenic plants that show the expression of desired DNA sequence specifically in guard cells, comprising the steps of:
a) producing an expression cassette which comprises the following sequences
i) a promoter, wherein said promoter comprises a nucleotide sequence of SEQ I.D. NO: 1, or a base sequence substantially having the nucleotide sequence of SEQ ID NO:1 which retains the promoter function of the base sequence of SEQ ID NO:1,
ii) a desired DNA fragment operably linked to said promoter, and
b) transferring said expression cassette into plant cells, thereby producing transformed plant cells and
c) regenerating whole transgenic plants from the transformed cells.

9. A method according to claim 8, wherein said promoter is isolated from *Arabidopsis thaliana* gene coding for trehalase.

10. A method according to claim 8, wherein said promoter is the 2.0 kb SalI/SmaI fragment fused to the GUS reporter gene contained in the plasmid pLES98062, deposited as KCTC 0664BP.

11. A recombinant plant cell wherein the expression of a desired protein is under control of said promoter of claim 1.

12. A transgenic plant according to claim 11, wherein said plant is a dicotyledonous plants.

13. A transgenic plant according to claim 11, wherein said plant is a monocotyledonous plants.

## Patentansprüche

1. Isolierter Promotor, der die spezifische Expression eines gewünschten DNA-Fragmentes nur in den Schließzellen der Spaltöffnungen und nicht in Mesophyll- oder Epidermiszellen von Blättern transformierter Pflanzen bewirkt, und der die in der Seq.-ID.-Nr. 1 gezeigte Basensequenz oder eine Basensequenz aufweist, die im Wesentlichen die Nukleotidsequenz von Seq.-ID.-Nr. 1 hat, welche die Promotorfunktion der Basensequenz von Seq.-ID.-Nr. 1 beibehält.

2. Promotor nach Anspruch 1, welcher eine 2,0 kb große Nukleotidsequenz mit den folgenden Eigenschaften aufweist, nämlich
i) sie ist aus *Arabidopsis thaliana* isoliert;
ii) sie hat Restriktionsstellen für BstSFI, BamHI, BsaWI, HindIII, XbaI, BgIII, EcoRI, BsrDI und Smal; und
iii) sie umfasst ein auf Hitzeschock reagierendes Element und zwei auf Stress reagierende Elemente.

3. Promotor nach Anspruch 1, welcher der 5'-transkriptionsregulierende Bereich eines Trehalase-codierenden Gens ist.

4. Klonierungsvektor, wobei der Promotor in dem als KCTC 0663BP hinterlegten Plasmid pLES98061 enthalten ist, mit einem Promotor nach Anspruch 1.

5. Expressionsvektor, umfassend einen Promotor nach Anspruch 1, wobei der Promotor mit dem GUS-Reportergen oder einem in dem als KCTC 0664BP hinterlegten Plasmid pLES98062 enthaltenen gewünschten Strukturgen verbunden ist.

6. Mikroorganismus, der das Plasmid nach Anspruch 4 enthält, wobei der Mikroorganismus der *E. coli* XL1 -Blue/pLES98061 -Stamm ist.

7. Mikroorganismus, der das chimäre Gen nach Anspruch 5 enthält, wobei der Mikroorganismus der *Agrobacterium tumefaciens* LBA4404/pLES98062-Stamm ist.

8. Verfahren zur Herstellung transgener Pflanzen, die die gewünschte DNA-Sequenz spezifisch in Schließzellen exprimieren, umfassend die Schritte:
a) Herstellen einer Expressions-Kassette, die die folgenden Sequenzen umfasst:
i) einen Promotor, der eine Nukleotidsequenz der Seq.-ID.-Nr. 1 oder eine Basensequenz aufweist, die im Wesentlichen die Nukleotidsequenz von Seq.-ID.-Nr. 1 hat, welche die Promotorfunktion der Basensequenz von Seq.-ID.-Nr. 1 beibehält.
ii) ein gewünschtes DNA-Fragment, das funktionsfähig an den Promotor gebunden ist, und
b) Überführen der Expressions-Kassette in Pflanzenzellen, wodurch man transformierte Pflanzenzellen erhält und
c) Regenerieren vollständiger transgener Pflanzen aus den transformierten Zellen.

9. Verfahren nach Anspruch 8, wobei der Promotor aus *Arabidopsis thaliana-*Gen isoliert ist, das Trehalase codiert.

10. Verfahren nach Anspruch 8, wobei der Promotor das 2,0 kb große Sall/Smal-Fragment ist, das an das GUS-Reportergen gebunden ist, welches in dem als KCTC 0664BP hinterlegten Plasmid pLES98062 enthalten ist.

11. Rekombinante Pflanzenzelle, wobei die Expression eines gewünschten Proteins unter der Kontrolle des Promotors nach Anspruch 1 steht.

12. Transgene Pflanze nach Anspruch 11, wobei die Pflanze eine zweikeimblättrige Pflanze ist.

13. Transgene Pflanze nach Anspruch 11, wobei die Pflanze eine einkeimblättrige Pflanze ist.

## Revendications

1. Un promoteur isolé, lequel conduit à une expression spécifique d'un fragment d'ADN désiré seulement dans des cellules stomatiques de garde de plantes et non pas dans des cellules mésophylles ou épidermiques de feuilles de plantes transformées, comprenant la séquence de base montrée dans ID SEQ NO: 1, ou une séquence de base ayant sensiblement la séquence de nucléotides de ID SEQ NO: 1 qui maintient la fonction de promoteur de la séquence de base de ID SEQ NO: 1.

2. Un promoteur selon la revendication 1, dans lequel ledit promoteur comprend une séquence de nucléotides de 2,0 Kb ayant les caractéristiques suivantes :
i) être isolé de *Arabidopsis thaliana*;
ii) avoir des sites d'enzymes de restriction pour BstSFI, BamHI, BsaWI, HindIII, XbaI, BgIII, EcoRI, BsrDI et SmaI ; et
iii) comprendre un élément sensible aux chocs thermiques et deux éléments sensibles aux contraintes.

3. Un promoteur selon la revendication 1, dans lequel ledit promoteur est la région régulatrice transcriptionnelle 5' d'un gène de codage pour la tréhalase.

4. Un vecteur de clonage, dans lequel ledit promoteur est contenu dans le plasmide pLES98061 et est déposé en tant que KCTC 0663BP, comprenant un promoteur tel que défini à la revendication 1.

5. Un vecteur d'expression comprenant un promoteur tel que défini à la revendication 1, dans lequel ledit promoteur est soudé au gène notificateur de GUS ou à un gène structurel désiré contenu dans le plasmide pLES98062, déposé en tant que KCTC 0664BP.

6. Un microorganisme contenant le plasmide selon la revendication 4, dans lequel ledit microorganisme est la souche E. coli XL1-Blue/pLES98061.

7. Un microorganisme contenant le gène chimérique selon la revendication 5, dans lequel ledit microorganisme est la souche *Agrobacterium tumefaciens* LBA4404/pLES98062.

8. Un procédé pour la production de plantes transgéniques qui présentent l'expression de séquence d'ADN désirée spécifiquement dans des cellules de garde, comprenant les étapes consistant à :
a) produire une cassette d'expression qui comprend les séquences suivantes
i) un promoteur, dans lequel ledit promoteur comprend une séquence de nucléotides de ID SEQ NO: 1, ou une séquence de base ayant sensiblement la séquence de nucléotides de ID SEQ NO: 1 qui maintient la fonction de promoteur de la séquence de base de ID SEQ NO: 1,
ii) un fragment d'ADN désiré relié de manière exploitable audit promoteur, et
b) transférer ladite cassette d'expression dans des cellules de plantes, en produisant ainsi des cellules de plantes transformées et
c) régénérer l'ensemble des plantes transgéniques à partir des cellules transformées.

9. Un procédé selon la revendication 8, dans lequel ledit promoteur est isolé du gène *Arabidopsis thaliana* de codage pour la tréhalase.

10. Un procédé selon la revendication 8, dans lequel ledit promoteur est le fragment de 2,0 kb SaII/SmaI soudé au gène de GUS contenu dans le plasmide pLES98062, déposé en tant que KCTC 0664BP.

11. Une cellule de plante recombinante dans laquelle l'expression d'une protéine désirée est sous la commande dudit promoteur de la revendication 1.

12. Une plante transgénique selon la revendication 11, dans laquelle ladite plante est une des plantes dicotylédones.

13. Une plante transgénique selon la revendication 11, dans laquelle ladite plante est une des plantes monocotylédones.


### (SEQUENCE LISTING)

<110> KOREA KUMGO PETROCHEMICAL CO., LTD.
<120> Promoter for guard cell-specific gene expression in plants
<130> P99-12-26
<160> 1
<170> KOPATIN 1.5
<210> 1
   <211> 2087
   <212> DNA
   <213> Arabidopsis thaliana
<220>
   <221> misc_feature
   <222> (1)..(1)
   <223> BstSFI cleavage site at 1
<220>
   <221> misc_feature
   <222> (2087)..(2087)
   <223> SmaI cleavage site at 2087
<220>
   <221> promoter
   <222> (1)..(2087)
   <223> guard cell specific promoter
<400> 1
   ctgcagcatt ttctcggttc aaaatcgtta ttcctgtaat atattgatcc atcccacgat 60
   cttcttttct tggtcgtctt ctgaaacgct aatcggatcc agaagccatg gacaaaagca 120
   tcagcatcac ctccaacgtt agcacgacga cttccatgga aaagatcgac cacgccgcta 180
   gctggatcag cgcaaccgta atatccgctt tcttcacctc cctcgaacgc tgcgcctgcg 240
   tcaatctctc cacctctcat gacgacgacg acgacgacga cgacgaatcc catcaccgtc 300
   ctctcgctct ctccgccgct cctcatccaa acgacaccgt ttagctctgt tttttaacct 360
   ctggtaattt ataaatttaa tccctcgttt gtttcttttg ataatcgatt ggtgtggatt 420
   tgtaatttct tgatttgaaa atgtatgtct actgtgtgta aatgtgcatg tctcagaatt 480
   tgattgtttt gcgtgtatct gactttatat acttgggttt aaaatcagaa tgaatcatcg 540
   tttttgcatt tacattgaaa aaatatcaag aagagaattt ggtagagatc atttgtttgt 600
   tttgaagttt tcttttgtga tcaatgtttc tcgatttacc tttttcattt catttctgat 660
   ctctgcgttc aactattaaa accggaacag catcagtttc atgttttatt ttgtcaatgt 720
   gtcagagatc ctgtcacttt agatttcata gtttcaaaag tgattatttg atcatgtggg 780
   tccctgctgt gagaaccagt gggcactggc ctttagtttg gttaaaagca ggattcaaaa 840
   cttcatacta gtttctttgt tctaaaatgt atttagcctt tttcaaactc aagttgcact 900
   tgttgtacaa aagacacttt tcttttgaat ctaatttaac attacattca aaaaaaaaaa 960
   aaacttcata ctaagtaaat gtcgtgtttg atccaaactt ttcccaattt gtttgcttag 1020
   cagggtgcaa tcttgtttaa atatctttgt catgtataat gtgattccat ctcttcatcc 1080
   atatagagaa aatcttgttt aaatgtcttt tgtcatatga ccatattctc acttgggttt 1140
   ctagaaaaag ttttgggttc tttgagtttg ttgaattgga tctccttatg gcttagaccg 1200
   tcgttccaac tgctgaagaa gcaagtatgt tgactcaaaa cagtcacgtt atatctctgt 1260
   ctctttgata attcttggct tttatgtgtt gggtgcctag tatcatttgt tttaaaggtt 1320
   tagggaacaa aaggtcataa actataataa gaatactttt tacaatgtta tttcctcagc 1380
   aaaagtagca cgcattcaca ttctccaagt ttcatcattt gttatttatg aacgcaaaca 1440
   aaaaaactgt actattaaca agcttttgtt aagaaaaaag taaggaaacc ctcttaggat 1500
   atgtatgttt caacaataag cttatatctt tactcattaa gcccactaga caactttgtg 1560
   agatagatct tataagaagc gtatcacttt atccccgtga ttacttctca aaacaacgag 1620
   ttttgatttg tgctacttta ttaaattcac gttctgtttt cagaaatttt gttttttctg 1680
   ataggttcgt attgaaattg acctctttgg aaactggatc tttcataaga tagaaagaga 1740
   ggtccccatt tcccaagttt atcaaacaaa aagaaaaaaa aatctaaatt gttctcatct 1800
   tgagacaacc aatcagaatg aaccatctga gtcagccaaa gggtttaata gtcaccattt 1860
   gccaattgta ttcatactct ttcctttttg gtgcgttatt ctaagagccc tagtcagaat 1920
   tcaaacattt ggcaatgaaa tcatacaaac ttaataaccc taatttactc atctccacgc    1980
   acacacataa taaactcttc ttatcctctt ctccattcaa tctcttattc tcttttcctt    2040
   ccttcatata ccttaaacag caacgttctc tgttcttctt ccccggg                   2087